# EUROPÄISCHE PATENTANMELDUNG

(11) **EP 3 090 708 A1**
(43) Veröffentlichungstag der Anmeldung: **09.11.2016**
(21) Anmeldenummer: 16164002.4
(22) Anmeldetag: 06.04.2016
(51) Int. Cl.: A61F 5/01

(54) **KNIEORTHESE ZUM AUFBRINGEN EINER VENTRAL ODER DORSAL GERICHTETEN TRANSLATIONSKRAFT**

(30) Priorität: 04.05.2015 DE 102015005572
(71) Anmelder: Albrecht GmbH, 83071 Stephanskirchen (DE)
(72) Erfinder: OPAHLE, Hans-Georg, 83024 Rosenheim (DE); KAHLES, Martin, 83128 Halfing (DE)
(74) Vertreter: Bauer, Friedrich

(57) **Zusammenfassung**

Bei einer Knieorthese zum Aufbringen einer ventral oder dorsal gerichteten Translationskraft (F) umfasst die Translationskraftaufbringungseinrichtung eine Seilzugeinrichtung (19) mit einer Seilkrafterzeugungseinrichtung (20) und einem Seilzug (21), der sich zumindest abschnittsweise von den Unterschenkelschienen (11a, 11b) nach dorsal oder ventral über einen proximalen Abschnitt des Unterschenkels (3) quer zur Längserstreckung der Unterschenkelschienen (11a, 11b) erstreckt, an den Unterschenkelschienen (11a, 11b) befestigt oder umgelenkt ist und mittels einer von der Seilkrafterzeugungseinrichtung (20) in den Seilzug (21) eingeleiteten Seilkraft den proximalen Abschnitt des Unterschenkels (3) in die ventrale oder dorsale Richtung drängt.

## Beschreibung

Die Erfindung betrifft eine Knieorthese zum Aufbringen einer ventral oder dorsal gerichteten Translationskraft auf einen proximalen Abschnitt eines Unterschenkels gemäß dem Oberbegriff des Patentanspruchs 1.

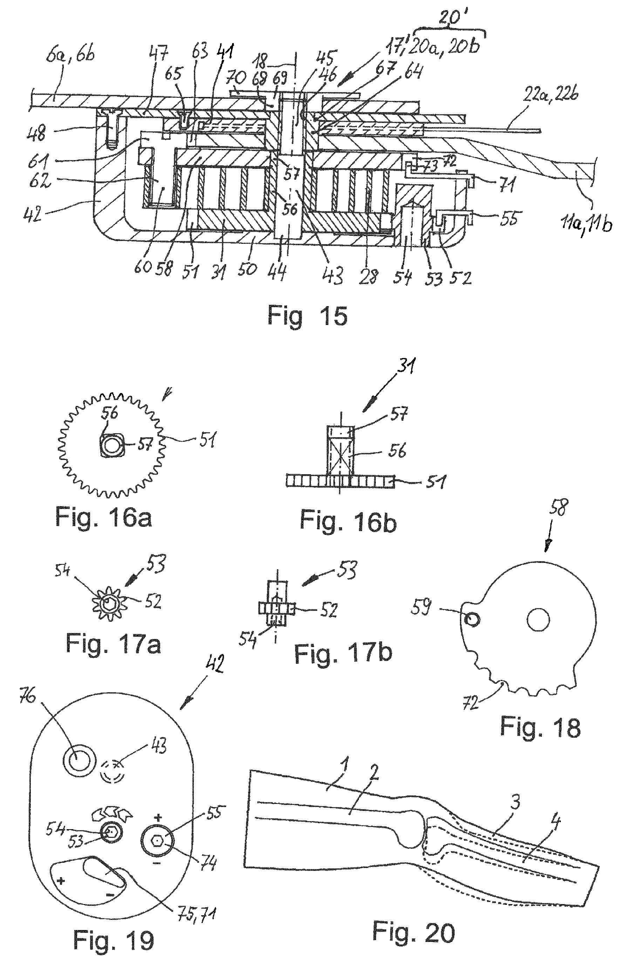

Auf dem Gebiet von Knieinstabilitäten bereiten insbesondere hintere Kreuzbandläsionen große medizinische Probleme. Obwohl es ausgereifte chirurgische Rekonstruktionsverfahren für hintere Kreuzbandrupturen und andere Läsionen der hinteren Kreuzbänder gibt, wird das operative Ergebnis häufig nach einigen Monaten wieder dadurch verschlechtert oder völlig zunichte gemacht, dass das genähte oder rekonstruierte Band beispielsweise aufgrund der Schwerkraft des Unterschenkels, die bei einem auf dem Rücken liegenden Patienten in dorsaler Richtung wirkt, oder aufgrund des Muskelzugs in unerwünschter Weise wieder gedehnt wird. Hierdurch kommt es zu einer Fehlstellung der Tibia zum Femur, wie in Figur 20 in gestrichelten Linien dargestellt ist. Die durchgezogenen Linien in Figur 20 zeigen dagegen die Tibia und den Unterschenkel im Normalzustand. Die Gefahr einer derartigen Fehlstellung, d.h. des Absenkens der Tibia relativ zum Femur, besteht selbstverständlich auch dann, wenn eine hintere Kreuzbandläsion nicht operativ, sondern konservativ behandelt wird.

Bei einer vorderen Kreuzbandinstabilität kann das Problem auftreten, dass die Tibia relativ zum Femur zu weit ventral zu liegen kommt. Dies ist insbesondere dann der Fall, wenn die Tibiagelenkfläche des Patienten so geneigt ist, dass bei aufrechtem Stand, bei dem das Femur auf die schräge Tibiagelenkfläche drückt, auf die Tibia eine Kraft in ventraler Richtung ausübt. Ist das vordere Kreuzband instabil, wird dadurch die Tibia im Kniegelenkbereich in unerwünschter Weise nach vorn, d.h. in Figur 20 nach oben verschoben.

Aus der Patentschrift EP 1 114 619 A1 ist bereits eine Vorrichtung zum Aufbringen einer ventral oder dorsal gerichteten Translationskraft auf den Unterschenkel im Bereich des Kniegelenks bekannt, die dieser unerwünschten Verlagerung der Tibia entgegenwirken soll. Diese bekannte Vorrichtung weist medial und lateral im Bereich des Oberschenkels angeordnete Oberschenkelschienen auf, die mittels einer Manschette am Oberschenkel befestigt werden können. Am distalen Ende der Oberschenkelschienen, die sich seitlich neben dem Knie befinden, ist ein L-förmiger Schwenkhebel gelenkig angebracht, welcher mittels einer Feder entweder in ventraler oder dorsaler Richtung vorgespannt ist. Dieser Schwenkhebel unter- oder übergreift einen seitlich von einer Unterschenkelmanschette abstehenden Bolzen in einem knienahen Bereich des Unterschenkels. Je nachdem, wie die Vorspannung der Feder gerichtet ist, wird hierdurch das obere Ende der Tibia entweder in ventraler oder dorsaler Richtung gedrängt, um einer unerwünschten Dehnung eines lädierten hinteren bzw. vorderen Kreuzbandes und einer damit verbundenen translatorischen Verschiebung der Tibia in dorsaler bzw. ventraler Richtung entgegenzuwirken. Problematisch ist bei dieser bekannten Vorrichtung jedoch, dass durch die Federkraft auf den Unterschenkel eine permanente Kraft in Extensions- bzw. Flexionsrichtung ausgeübt wird, d.h. ein Drehmoment, welches den Unterschenkel permanent zu strecken bzw. zu beugen versucht. Ein freies, ungehindertes Strecken bzw. Beugen des Knies ist daher mit dieser bekannten Vorrichtung nicht möglich.

Aus der WO 2004/056293 A1 ist eine Knieorthese gemäß dem Oberbegriff des Anspruchs 1 bekannt, bei der im Bereich des Unterschenkels ein längerer und ein kürzerer Schienenarm vorgesehen sind, die relativ zueinander schwenkbar sind. Der kürzere Schienenarm ist mit seinem distalen Ende an einer schalenartigen Unterschenkelbefestigungseinrichtung in einem knienahen Bereich angelenkt und mittels einer im Bereich der Gelenkeinrichtung angeordneten Feder ventral oder dorsal vorgespannt, wodurch auf den knienahen Bereich des Unterschenkels eine nach ventral oder dorsal gerichtete Translationskraft unabhängig vom Extensions- bzw. Flexionswinkel des Kniegelenks ausgeübt wird.

Ausgehend von diesem Stand der Technik liegt der Erfindung die Aufgabe zugrunde, eine alternative Knieorthese zum Aufbringen einer ventral oder dorsal gerichteten Translationskraft in einem knienahen Bereich des Unterschenkels zu schaffen, die auf einfache und effektive Weise die gewünschte Translationskraft erzeugt.

Diese Aufgabe wird erfindungsgemäß durch eine Knieorthese mit den Merkmalen des Anspruchs 1 gelöst. Vorteilhafte Ausführungsformen der Erfindung sind in den weiteren Ansprüchen beschrieben.

Erfindungsgemäß umfasst die Translationskraftaufbringungseinrichtung eine Seilzugeinrichtung mit einer Seilkrafterzeugungseinrichtung und einem Seilzug, der sich zumindest abschnittsweise von den Unterschenkelschienen nach dorsal oder ventral über einen proximalen Abschnitt des Unterschenkels quer zur Längserstreckung der Unterschenkelschienen erstreckt, an den Unterschenkelschienen befestigt oder umgelenkt ist und mittels einer von der Seilkrafterzeugungseinrichtung in den Seilzug eingeleiteten Seilkraft den proximalen Abschnitt des Unterschenkels in die ventrale oder dorsale Richtung drängt.

Für die Erfindung ist es somit charakteristisch, dass mittels eines Seilzugs auf den proximalen Abschnitt des Unterschenkels eine nach ventral oder dorsal gerichtete Kraft eingeleitet wird. Die in die entgegengesetzte Richtung wirkende Seilreaktionskraft wird dagegen in die Unterschenkelschienen eingeleitet. Zu diesem Zweck kann der Seilzug gemäß einer ersten Ausführungsform direkt an den Unterschenkelschienen befestigt sein. In diesem Fall ist es zweckmäßig, wenn die Seilzugerzeugungseinrichtung zumindest annähernd auf derjenigen Höhe des Unterschenkels angeordnet ist, auf welcher der Seilzug verläuft. Gemäß einer zweiten Ausführungsform ist es auch möglich, die Seilkrafterzeugungseinrichtung an anderer Stelle anzuordnen, insbesondere im Bereich der Gelenkeinrichtung. In diesem Fall wird der Seilzug von der Seilkrafterzeugungseinrichtung zunächst bis zu derjenigen Stelle der Unterschenkelschienen geführt, die zumindest annähernd auf derjenigen Höhe des Unterschenkels liegt, auf der die Translationskraft eingeleitet werden soll, worauf der Seilzug dann mittels einer entsprechenden Seilumlenkreinrichtung quer zu den Unterschenkelschienen nach dorsal bzw. ventral umgelenkt wird.

Mit Hilfe der Erfindung kann auf besonders wirkungsvolle und einfache Weise eine ventral oder dorsal gerichtete Translationskraft auf den proximalen, d.h. knienahen Abschnitt des Unterschenkels aufgebracht werden. Darüber hinaus bietet die Erfindung aufgrund der Verwendung eines Seilzugs eine große konstruktive Flexibilität und ermöglicht eine unauffällig konstruktive Lösung zum Aufbringen der Translationskraft.

Vorteilhafterweise umfasst der Seilzug zwei Zugseile, wobei ein erstes Zugseil an der lateralen Unterschenkelschiene und ein zweites Zugseil an der medialen Unterschenkelschiene befestigt ist, und die Seilkrafterzeugungseinrichtung ein zentrales Seilkrafterzeugungsmittel ist, mit der beide Zugseile in Wirkverbindung sind. Bei dieser Ausführungsform wird somit ein einziges Seilkrafterzeugungsmittel verwendet, mit dem beide Zugseile in Wirkverbindung stehen, wodurch eine besonders einfache und kostengünstige Lösung geschaffen werden kann. Vorteilhafterweise ist hierbei das zentrale Seilkrafterzeugungsmittel mittig zwischen der medialen und lateralen Unterschenkelschiene im dorsalen bzw. ventralen Bereich des Unterschenkels angeordnet.

Vorteilhafterweise ist die Seilkrafterzeugungseinrichtung auf einem zur Anlage am Unterschenkel bestimmten und relativ zu den Unterschenkelschienen bewegbaren Schalenteil befestigt. Besonders vorteilhaft ist hierbei, wenn das Schalenteil ein proximaler Teilabschnitt der Unterschenkelbefestigungseinrichtung ist, der relativ zu den Unterschenkelschienen in dorsaler und ventraler Richtung schwenkbar ist. Alternativ hierzu ist es jedoch auch denkbar, das zur Befestigung der Seilkrafterzeugungseinrichtung dienende Schalenteil getrennt von der Unterschenkelbefestigungseinrichtung auszubilden.

Gemäß einer alternativen Ausführungsform umfasst der Seilzug mindestens ein Zugseil, das einen ersten Zugseilabschnitt, der längs einer Unterschenkelschiene verläuft, und einen zweiten Zugseilabschnitt aufweist, der sich quer zu dieser Unterschenkelschiene nach dorsal oder ventral über den Unterschenkel erstreckt und mittels einer an der Unterschenkelschiene angeordneten Seilumlenkeinrichtung umgeleitet wird, wobei die Seilkrafterzeugungseinrichtung im Bereich der Gelenkeinrichtung oder Unterschenkelschiene angeordnet ist.

Besonders vorteilhaft ist es hierbei, wenn der Seilzug ein erstes Zugseil und ein zweites Zugseil umfasst, wobei das erste Zugseil einen im Bereich der lateralen Unterschenkelschiene angeordneten ersten Zugseilabschnitt und das zweite Zugseil einen im Bereich der medialen Unterschenkelschiene angeordneten ersten Zugseilabschnitt aufweisen, und wobei die Seilkrafterzeugungseinrichtung ein erstes Seilkrafterzeugungsmittel, das lateral im Bereich der Gelenkeinrichtung oder der lateralen Unterschenkelschiene angeordnet ist, und ein zweites Seilkrafterzeugungsmittel umfasst, das medial im Bereich der Gelenkeinrichtung oder der medialen Unterschenkelschiene angeordnet ist und ein zweites Seilkrafterzeugungsmittel umfasst, das medial im Bereich der Gelenkreinrichtung oder der medialen Unterschenkelschiene angeordnet ist. Hierdurch können sowohl lateral als auch medial entsprechende Seilkräfte erzeugt und zur gewünschten Stelle am Unterschenkel übertragen werden. Die Seilkrafterzeugung und -einleitung kann hierdurch relativ zu den Unterschenkelschienen sehr symmetrisch erfolgen. Da weiterhin zwei Seilkrafterzeugungsmittel vorgesehen sind, können diese schwächer und damit kleiner ausgeführt werden als bei Verwendung eines einzigen Seilkrafterzeugungsmittels. Alternativ können bei gleicher Baugröße größere Seilkräfte erzeugt werden.

Auch bei dieser Ausführungsform können sich die zweiten Zugseilabschnitte über ein zur Anlage am Unterschenkel bestimmtes und relativ zu den Unterschenkelschienen bewegbares Schalenteil erstrecken. Besonders vorteilhaft ist es hierbei, wenn das Schalenteil ein proximaler Teilabschnitt der Unterschenkelbefestigungseinrichtung ist, der relativ zu den Unterschenkelschienen nach dorsal und ventral schwenkbar ist.

Gemäß einer vorteilhaften Ausführungsform umfasst die Seilkrafterzeugungseinrichtung eine drehbare Seilscheibe, an der das mindestens eine Zugseil außermittig befestigt ist, wobei die Seilscheibe mittels einer Federeinrichtung in eine bestimmte Drehrichtung vorgespannt ist. Mittels einer derartigen Seilkrafterzeugungseinrichtung kann eine sehr kompakte Bauweise erzielt werden.

Vorteilhafterweise umfasst die Federeinrichtung eine Spiralfeder, die einen ersten Endbereich, der mit einem Einstellzahnrad gekoppelt ist, und einen zweiten Endbereich aufweist, der mit der Seilscheibe gekoppelt ist, wobei die Vorspannung der Seilscheibe durch Drehen des Einstellzahnrads variierbar ist. Hierdurch kann auf sehr einfache Weise die Vorspannung der Seilscheibe und damit die nach dorsal oder ventral wirkende Translationskraft eingestellt bzw. verändert werden.

Die Erfindung wird nachfolgend anhand der Zeichnungen beispielshaft näher erläutert. Es zeigen:
- Figur 1:: eine Seitenansicht eines Beins und einer etwas vereinfacht dargestellten Knieorthese gemäß einer ersten Ausführungsform der Erfindung;
- Figur 2:: das Bein und die Knieorthese von Figur 1 von hinten;
- Figur 3:: eine Darstellung gemäß Figur 2, wobei die Seilkrafterzeugungseinrichtung ohne Gehäuse dargestellt ist;
- Figur 4:: eine etwas vereinfachte Seitendarstellung der in den Figuren 1 bis 3 dargestellten Seilkrafterzeugungseinrichtung;

- Figur 5:: eine Draufsicht auf die Seilscheibe mit daran befestigten Zugseilen;
- Figur 6:: eine Draufsicht auf die Seilscheibe von Figur 5 in Alleinstellung;
- Figur 7:: einen Schnitt längs der Linie VII-VII von Figur 6;
- Figur 8:: eine Draufsicht auf eine in der Seilkrafterzeugungseinrichtung verwendete Spiralfeder;
- Figur 9:: eine Seitenansicht eines Beines und einer etwas vereinfacht dargestellten Knieorthese gemäß einer zweiten Ausführungsform der Erfindung;
- Figur 10:: eine Ansicht von hinten des Beines und der Knieorthese von Figur 9;
- Figur 11:: eine Seitenansicht entsprechend Figur 9 ohne Gehäuse der Gelenkeinrichtung/Seilkrafterzeugungseinrichtung, um die Seilscheibe erkennen zu können;
- Figur 12:: eine Draufsicht auf die Seilscheibe mit daran befestigtem Seilzug;
- Figur 13:: die Seilscheibe von Figur 12 in Alleinstellung ohne Seilzug;

- Figur 14:: eine Draufsicht auf eine Abdeckplatte, des Gehäuses;
- Figur 15:: einen teilweise schematisch dargestellten Schnitt durch die Gelenkeinrichtung/Seilkrafterzeugungseinrichtung der zweiten Ausführungsform;
- Figuren 16a, 16b:: eine Draufsicht bzw. Seitenansicht eines zentralen Zahnrads;
- Figuren 17a, 17b:: eine Draufsicht bzw. Seitenansicht eines Antriebszahnrads;
- Figur 18:: eine Draufsicht auf eine Mitnehmerscheibe;
- Figur 19:: eine Draufsicht auf das Gehäuse der Gelenkeinrichtung/Seilkraftumlenkeinrichtung;
- Figur 20:: eine schematische Darstellung des Beins eines Menschen im Bereich des Kniegelenks mit Femur und Tibia, wobei Unterschenkel und Tibia in Normalstellung und einer dorsal verschobenen Stellung dargestellt sind.

Aus Figur 20 ist ein Oberschenkel 1 mit einem Femur 2 sowie ein Unterschenkel 3 mit einer Tibia 4 ersichtlich. Hierbei ist mit durchgezogener Linie die Normallage und mit gestrichelten Linien eine dorsal abgesenkte Lage des Unterschenkels 3 und der Tibia 4 dargestellt, die bei Kreuzbandinstabilitäten aufgrund der dorsal wirkenden Schwerkraft bei einem auf dem Rücken liegenden Patienten oder aufgrund des Muskelzugs entstehen kann.

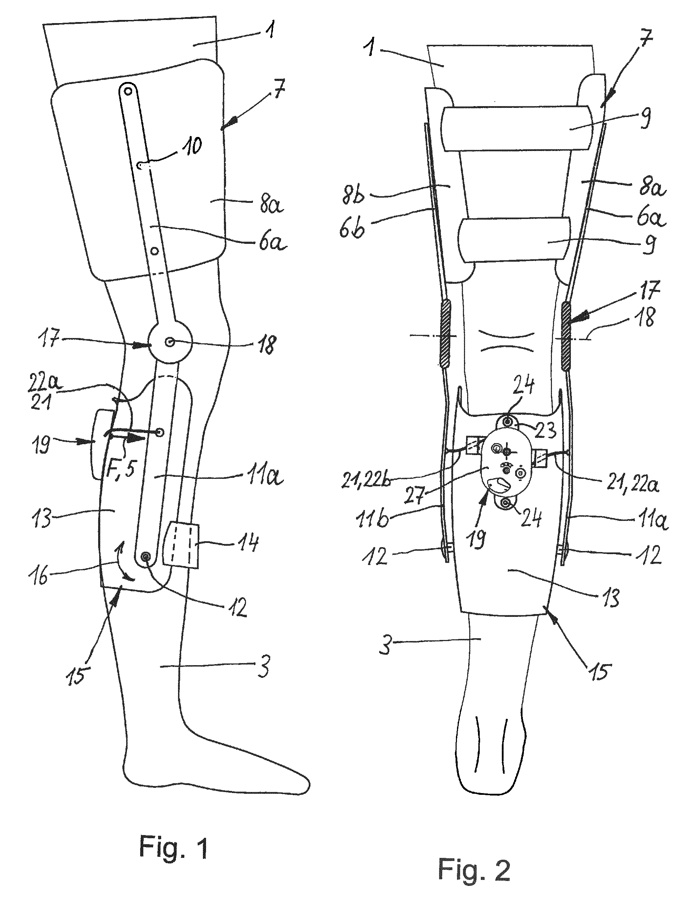

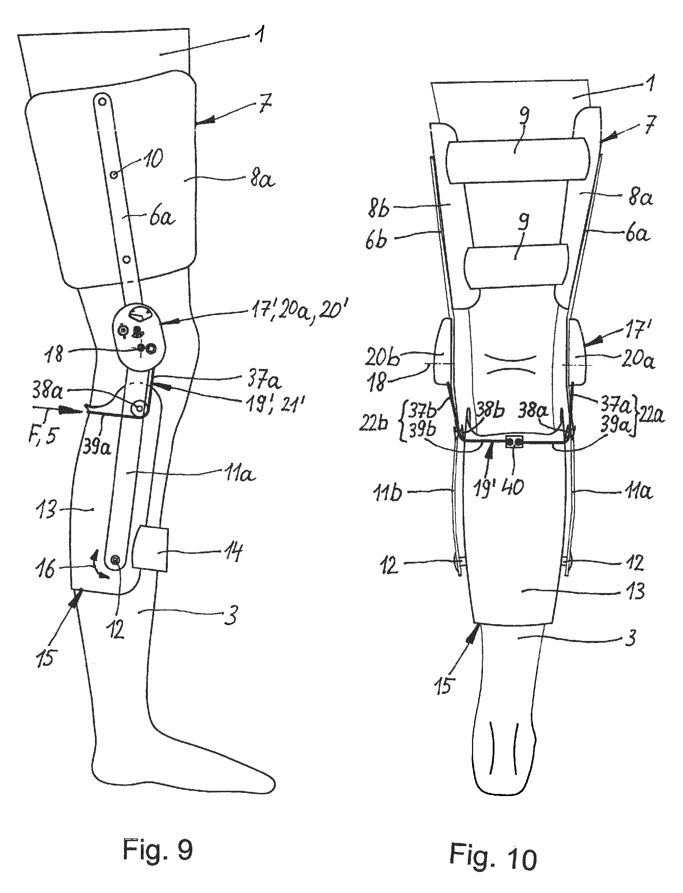

Anhand der Figuren 1 bis 8 wird im Folgenden eine erste Ausführungsform einer Knieorthese beschrieben, mit der auf den Unterschenkel 3 in einem proximalen (knienahen) Abschnitt eine der Tibiaabsenkung entgegenwirkende, ventral gerichtete Translationskraft F aufgebracht werden kann, um eine Absenkung der Tibia 4 auch bei geschwächten, verletzten oder rekonstruierten Kreuzbändern von vorne herein nicht entstehen zu lassen oder eine Absenkung rückgängig zu machen. Die Figuren 9 bis 19 zeigen eine zweite Ausführungsform einer derartigen Knieorthese. Die Richtung der durch diese Knieorthesen aufgebrachten Translationskraft F ist hierbei in den Figuren 1 und 9 mit dem Pfeil 5 gekennzeichnet.

Die in den Figuren 1 bis 3 dargestellte Knieorthese umfasst Oberschenkelschienen 6a, 6b, die lateral bzw. medial am Oberschenkel 1 anordenbar und mittels einer Oberschenkelbefestigungseinrichtung 7 am Oberschenkel 1 befestigbar sind. Bei der Oberschenkelbefestigungseinrichtung 7 kann es sich beispielsweise um laterale und mediale Schalenteile 8a, 8b handeln, die mittels Haltebändern 9 mit Klettverschluss zusammengehalten werden. Die Schalenteile 8a, 8b sind dabei beispielsweise mittels Schrauben oder Nieten 10 fest mit den Oberschenkelschienen 6a, 6b verbunden.

Die Knieorthese umfasst ferner Unterschenkelschienen 11a, 11b, die lateral bzw. medial am Unterschenkel 3 anordenbar sind. Die Unterschenkelschienen 11a, 11b sind in ihrem distalen Endbereich mittels Gelenkzapfen 12 an einem Schalenteil 13 in Form einer Halbschale angelenkt. Beim Schalenteil 13 kann es sich beispielsweise um ein relativ formstabiles Kunststoffteil handeln, das sich etwas über mehr als 180° über den dorsalen (posterioren), lateralen und medialen Bereich des Unterschenkels 3 erstreckt. In Längsrichtung des Unterschenkels 3 erstreckt sich das Schalenteil 13 bis in einen knienahen Bereich. Weiterhin ist das Schalenteil 13 in einem Bereich, der nahe den Gelenkzapfen 12 liegt, mittels eines Haltebandes 14, insbesondere mit Klettverschluss, am Unterschenkel 3 befestigbar.

Das Schalenteil 13 und das Halteband 14 sind somit Teile einer Unterschenkelbefestigungseinrichtung 15, mit welcher der distale Endbereich der Unterschenkelschienen 11a, 11b am Unterschenkel 3 befestigbar ist. Das Schalenteil 13 kann dabei um die Gelenkzapfen 12 herum geschwenkt werden, wie durch den Doppelpfeil 16 in Figur 1 dargestellt. Insbesondere lässt sich hierdurch der proximale Endbereich des Schalenteils 13 relativ zu den Unterschenkelschienen 11a, 11b frei nach Ventral bzw. Dorsal bewegen.

Die Unterschenkelschienen 11a, 11b sind mittels einer üblichen Gelenkeinrichtung 17 gelenkig mit den Oberschenkelschienen 6a, 6b verbunden und um eine Schwenkachse 18 schwenkbar, um eine Flexion und Extension des Knies zu ermöglichen. In den Figuren 1 bis 3 ist diese Gelenkeinrichtung 17 lediglich schematisch dargestellt. Die Gelenkeinrichtung 17 kann derart beschaffen sein, dass die Flexion und Extension des Knies innerhalb vorgegebener Schwenkbereichsgrenzen frei, das heißt ohne Einfluss einer in Extensions- oder Flexionsrichtung wirkenden Federkraft, möglich ist. Alternativ kann die Gelenkeinrichtung 17 auch eine Feder enthalten, die eine Vorspannkraft in Extensions- oder Flexionsrichtung ausübt.

Um auf den proximalen Abschnitt des Unterschenkels 3 eine ventral gerichtete Translationskraft F aufzubringen, umfasst die Knieorthese ferner eine Translationskraftaufbringungseinrichtung in Form einer Seilzugeinrichtung 19 mit einer Seilkrafterzeugungseinrichtung 20 und einem Seilzug 21, der zwei Zugseile 22a, 22b umfasst.

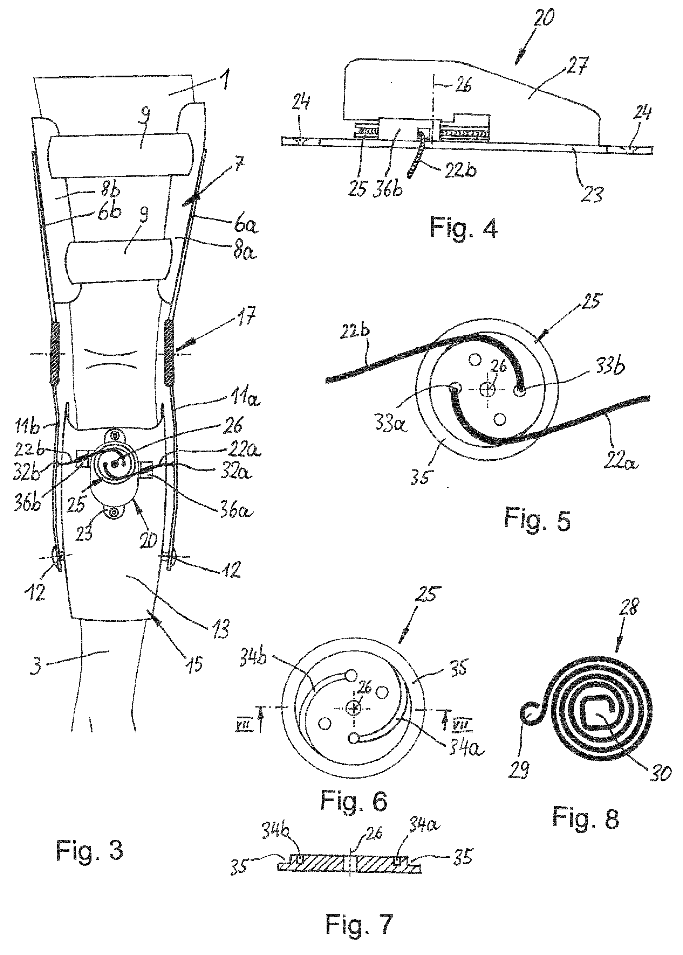

Wie aus den Figuren 1 bis 3 ersichtlich, ist die Seilkrafterzeugungseinrichtung 20 auf einem proximalen Abschnitt des Schalenteils 13 befestigt. Die Befestigung erfolgt über eine Basisplatte 23 (Figur 4) die mittels Schrauben 24 (Figur 2) am Schalenteil 13 festgeschraubt wird. In der rückseitigen Ansicht von Figur 2 befindet sich die Seilkrafterzeugungseinrichtung 20 mittig zwischen den Unterschenkelschienen 11a, 11b.

Die Seilkrafterzeugungseinrichtung 20 umfasst eine in den Figuren 3 bis 7 dargestellte Seilscheibe 25, die um eine Drehachse 26 drehbar ist. Die Seilscheibe 25 ist innerhalb eines auf der Basisplatte 23 befestigten Gehäuses 27 der Seilkrafterzeugungseinrichtung 20 drehbar gelagert.

Die Seilkrafterzeugungseinrichtung 20 umfasst ferner eine in Figur 8 dargestellte Spiralfeder 28, die oberhalb der Seilscheibe 25 mittig zur Drehachse 26 angeordnet ist. Ein äußerer Endbereich 29 der Spiralfeder 28 ist dabei über einen nicht dargestellten Kopplungsstift, gegebenenfalls unter Zwischenschaltung einer anhand der Figuren 15 und 18 beschriebenen, jedoch mit der Seilscheibe 25 fest verbundenen Mitnehmerscheibe 58, mit der Seilscheibe 25 verbunden, während ein innerer Endbereich 30 der Spiralfeder 28 drehfest innerhalb des Gehäuses 27 fixiert ist oder mittels eines Einstellzahnrads 31 (Figuren 16a, 16b), Antriebszahnrads 17a, 17b (Figuren 17a, 17b) und einer Verriegelungseinrichtung 55 relativ zum Gehäuse 27 arretiert werden kann. Eine Vorspannung der Spiralfeder 28 in Umdrehungsrichtung der Seilscheibe 25 wird somit über den äußeren Endbereich 29 der Spiralfeder 28 auf die Seilscheibe 25 übertragen. Diese Vorspannung ist so gerichtet, dass in der in Figur 3 ersichtlichen Anordnung die Seilscheibe 25 im Uhrzeigersinn vorgespannt ist, das heißt die Spiralfeder 28 versucht, die Seilscheibe 25 um die Drehachse 26 im Uhrzeigersinn herum zu drehen.

Um die Vorspannung der Spiralfeder 28 einstellen zu können, kann ihr innerer Endbereich 30 mit dem ebenfalls um die Drehachse 26 herum drehbaren Einstellzahnrad 31 drehfest gekoppelt sein, das in den Figuren 16a, 16b dargestellt ist und später noch im Zusammenhang mit der zweiten Ausführungsform der erfindungsgemäßen Knieorthese beschrieben wird.

Das Zugseil 22a ist mit einem Ende 32a an der lateralen Unterschenkelschiene 11a befestigt. In gleicher Weise ist das Zugseil 22b mit einem Ende 32b an der medialen Unterschenkelschiene 11b befestigt. Diese Befestigungsstellen befinden sich auf einer Höhe der Unterschenkelschienen 11a, 11b, die in der proximalen Hälfte der Halbschale 13 liegt. Von diesen Befestigungsstellen erstrecken sich die Zugseile 22a, 22b quer zur Längserstreckung der Unterschenkelschienen 11a, 11b nach Dorsal zur Seilscheibe 25, an der sie mit ihren Enden 33a, 33b (Figur 5) auf gegenüberliegenden Seiten der Drehachse 26 außermittig befestigt sind. Die Zugseile 22a, 22b werden dabei in bogenförmigen Nuten 34a, 34b (Figuren 6, 7) von ihren Befestigungspunkten zu einer äußeren Umfangsnut 35 der Seilscheibe 25 und von dort über Seilführungen 36a, 36b aus der Seilkrafterzeugungseinrichtung 20 nach verschiedenen Seiten herausgeführt. Die Kopplung der Zugseile 22a, 22b mit der Seilscheibe 25 ist somit derart, dass durch die im Uhrzeigersinn auf die Seilscheibe 25 einwirkende Federvorspannkraft eine Zugkraft auf die Zugseile 22a, 22b aufgebracht wird. Diese Zugkraft bewirkt, dass die Seilkrafterzeugungseinrichtung 20 und damit der proximale Abschnitt der Halbschale 13 nach Ventral gedrängt werden, das heißt auf die Halbschale 13 und damit auf den Unterschenkel 3 wird eine Translationskraft F ausgeübt, die in Richtung des Pfeils 5 (Figur 1) wirkt und den proximalen Abschnitt der Halbschale 13 relativ zu den Unterschenkelschienen 11a, 11b in die ventrale Richtung schwenkt.

Anhand der Figuren 9 bis 19 wird im Folgenden eine zweite Ausführungsform der erfindungsgemäßen Knieorthese beschrieben. Die Wirkung der zweiten Ausführungsform auf den Unterschenkel 3 sowie die Anordnung des Schalenteils 13 sowie die schwenkbare Lagerung am distalen Ende der Unterschenkelschienen 11a, 11b ist gleich wie bei der ersten Ausführungsform, so dass insoweit hierauf Bezug genommen wird.

Wie aus den Figuren 9 bis 11 ersichtlich, umfasst die zweite Ausführungsform wiederum eine Translationskraftaufbringungseinrichtung in Form einer Seilzugeinrichtung 19' mit einer Seilkrafterzeugungseinrichtung 20' und einem Seilzug 21', der zwei Zugseile 22a, 22b umfasst.

Im Gegensatz zu der ersten Ausführungsform ist die Seilkrafterzeugungseinrichtung 20' der zweiten Ausführungsform nicht in Form eines zentralen Seilkrafterzeugungsmittels ausgebildet, das am Schalenteil 13 befestigt ist, sondern weist ein laterales Seilkrafterzeugungsmittel 20a und ein mediales Seilkrafterzeugungsmittel 20b auf, die im Bereich der Gelenkeinrichtung 17' angeordnet sind. Die Wirkung dieser Seilkrafterzeugungsmittel 20a, 20b auf den Unterschenkel 3 ist identisch zur Seilkrafterzeugungseinrichtung 20 der ersten Ausführungsform.

Die zwei Zugseile 22a, 22b der zweiten Ausführungsform erstrecken sich wiederum von den Seilkrafterzeugungsmitteln 20a, 20b bis in den dorsalen Bereich des Schalenteils 13. Hierzu weist das laterale Zugseil 22a einen ersten Zugseilabschnitt 37a auf, der vom lateralen Seilkrafterzeugungsmittel 20a längs der Unterschenkelschiene 11a bis zu einer Umlenkeinrichtung 38a verläuft. Die Umlenkeinrichtung 38a lenkt das Zugseil 22a in eine Richtung um, die quer zur Längserstreckung der Unterschenkelschiene 11a verläuft. Von der Umlenkeinrichtung 38a erstreckt sich somit ein zweiter Zugseilabschnitt 39a in dorsaler Richtung, um endseitig im dorsalen Bereich der Halbschale 13 mittels eines geeigneten Befestigungsmittels 40 im proximalen Endbereich der Halbschale befestigt zu werden.

In gleicher Weise erstreckt sich auf der medialen Seite ein erster Zugseilabschnitt 37b vom medialen Seilkrafterzeugungsmittel 20b längs der Unterschenkelschiene 11b in distaler Richtung bis zu einer medialen Umlenkeinrichtung 38b. Die Umlenkeinrichtung 38b lenkt den Seilzug 22b derart um, dass ein zweiter Zugseilabschnitt 39b quer zur Unterschenkelschiene 11b in dorsaler Richtung verläuft. Das Ende des zweiten Zugseilabschnittes 39b ist wiederum mittels des Befestigungsmittels 40 an der Halbschale 13 befestigt.

Die Umlenkeinrichtungen 38a, 38b sind an den Unterschenkelschienen 11a, 11b angeordnet und können anstelle der lediglich exemplarisch dargestellten Seilrollen auch aus anderen Mitteln bestehen, beispielsweise aus gebogenen Führungshülsen.

Wird mittels der Seilkrafterzeugungsmittel 20a, 20b eine Zugkraft auf die Zugseile 22a, 22b aufgebracht, wird der proximale Abschnitt der Halbschale 13 in gleicher Weise wie bei der ersten Ausführungsform in Richtung des Pfeils 5 geschwenkt, wobei eine ventral gerichtete Translationskraft F auf den proximalen Abschnitt des Unterschenkels 3 aufgebracht wird.

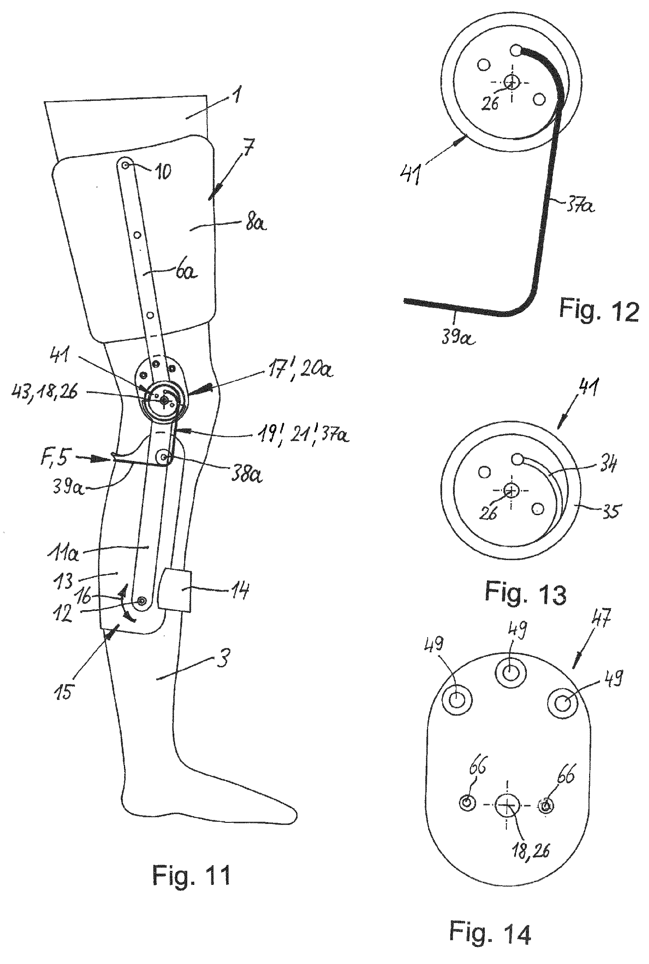

Zur Erzeugung der Seil- oder Zugkraft weisen die Seilkrafterzeugungsmittel 20a, 20b in gleicher Weise wie die Seilkrafterzeugungseinrichtung 20 der ersten Ausführungsform jeweils eine Seilscheibe 41 auf, die im Wesentlichen gleich aufgebaut ist und funktioniert wie die Seilscheibe 25 der ersten Ausführungsform. Unterschiedlich ist lediglich, dass jede der beiden Seilscheiben 41 lediglich mit einem Seilzug 22a bzw. 22b verbunden ist. Es ist daher ausreichend, wie in Figur 13 gezeigt, dass die Seilscheiben 41 jeweils nur eine bogenförmige Nut 34 aufweisen.

Jede Seilscheibe 41 ist wiederum in einer bestimmten Drehrichtung mittels einer Spiralfeder 28 vorgespannt, die identisch zu der in Figur 8 dargestellten und beschriebenen Spiralfeder 28 sein kann. Die Vorspannkraft der Spiralfeder 28 wirkt dabei auf die in Figur 11 gezeigte Seilscheibe 41 derart ein, dass diese eine Drehbewegung im Gegenuhrzeigersinn auszuführen versucht und dabei den ersten Zugseilabschnitt 37a nach oben zieht, was zu einer Bewegung des zweiten Zugseilabschnitts 39a in die ventrale Richtung und damit zu einer ventral gerichteten Translationskraft F auf den proximalen Abschnitt des Unterschenkels 3 führt.

Im Folgenden werden anhand der Figuren 14 bis 19 der Aufbau und die Funktionsweise der Seilkrafterzeugungsmittel 20a, 20b näher beschrieben. Beide Seilkrafterzeugungsmittel 20a, 20b sind grundsätzlich gleich, jedoch zueinander seitenverkehrt ausgebildet.

Wie aus Figur 15 ersichtlich, sind die Oberschenkelschiene 6a, 6b und die Unterschenkelschiene 11a, 11b mittels einer Gelenkeinrichtung 17' gelenkig miteinander verbunden und um die Schwenkachse 18 drehbar. Die Schwenkachse 18 wird durch einen zentralen, zylindrischen Lagerbolzen gebildet, der mit einem Ende 44 fest in einem napfförmigen Gehäuse 42 verankert, beispielsweise eingegossen, ist. Ein zum gegenüberliegenden freien Ende benachbarter Gewindeabschnitt 45 weist ein Außengewinde 46 auf.

Das Gehäuse 42 ist mit einer ebenen Abdeckplatte 47 weitgehend verschlossen, wobei die Abdeckplatte 47 mittels Schrauben 48 am Gehäuse 42 festgeschraubt ist.

Figur 14 zeigt eine Draufsicht auf die Abdeckplatte 47. Die drei Bohrungen zum Hindurchführen der drei Schrauben 48 sind mit 49 bezeichnet.

Wie aus Figur 15 weiter ersichtlich, ist auf dem Lagerbolzen 43 ein Einstellzahnrad 31 drehbar gelagert. Dieses Einstellzahnrad 31 ist in axialer Richtung durch eine Wand 50 des Gehäuses 42 abgestützt.

Die Verzahnung 51 des Einstellzahnrads 31 kämmt mit einer Verzahnung 52 eines kleineren Antriebszahnrads 53, das drehbar im Gehäuse 42 gelagert ist. Dieses Antriebszahnrad 53 dient dazu, die Drehposition des Einstellzahnrads 31 relativ zum Gehäuse 42 einzustellen. Hierzu weist das Antriebszahnrad 53 von außen zugängliche Werkzeugansatzflächen, beispielsweise in Form einer Sechskantaussparung 54, zum Einführen eines Inbusschlüssels auf, so dass das Antriebszahnrad 53 manuell mittels eines passenden Werkzeugs gedreht werden kann. Die Drehposition des Antriebszahnrads 53 und damit des Einstellzahnrads 31 kann mittels einer in Figur 15 lediglich schematisch eingezeichneten Verriegelungseinrichtung 55 verriegelt werden, die manuell in und außer Verriegelungseingriff mit der Verzahnung 52 des Antriebszahnrads 53 gebracht werden kann.

Wie aus den Figuren 15, 16a, 16b weiter ersichtlich, weist das Einstellzahnrad 31 ein zentral über die kreisförmige Zahnscheibe vorragenden Vierkantabschnitt 56 auf, auf dem ein zylindrischer Hülsenabschnitt 57 angeordnet ist. Auf den Vierkantabschnitt 56 ist die in Figur 8 dargestellte Spiralfeder 28 mit ihrem inneren Endbereich 30 drehfest aufgesteckt.

Auf dem Hülsenabschnitt 57 des Einstellzahnrads 31 ist eine Mitnehmerscheibe 58 drehbar gelagert, die in Figur 18 dargestellt ist. Die Mitnehmerscheibe 58 weist in einem umfangsnahen Bereich eine Gewindebohrung 59 auf, in die eine Mitnehmerschraube 60 eingeschraubt ist. Wie aus Figur 15 ersichtlich, steht der Schraubenkopf 61 dieser Mitnehmerschraube 60 nach oben über die Mitnehmerscheibe 60 vor, während der Schraubenschaft 62 auf der gegenüberliegenden Seite nach unten über die Mitnehmerscheibe 58 vorsteht und mit dem äußeren Endbereich 29 der Spiralfeder 28 gekoppelt ist.

Der Schraubenkopf 61 der Mitnehmerschraube 60 dient als Anschlag für eine Mitnehmernase 63 der Unterschenkelschiene 11a, 11b. Diese Mitnehmernase 63 ist im proximalen Endbereich der Unterschenkelschiene 11a, 11b angeordnet und hintergreift in einer in Figur 15 durch den Schraubenkopf 61 verdeckten Weise den Schraubenkopf 61.

Die Unterschenkelschiene 11a, 11b ist an einer zylindrischen Schraubhülse 64 drehbar gelagert, die auf den Gewindeabschnitt 45 des Lagerbolzens 43 aufgeschraubt ist.

Wie aus Figur 15 weiter ersichtlich, dient die Schraubhülse 64 auch zur Drehlagerung der Seilscheibe 41, die in den Figuren 12 und 13 dargestellt ist. Diese Seilscheibe 41 ist mittels Schrauben 65 fest mit der Abdeckplatte 47 verschraubt. In Figur 14 sind die Bohrungen zum Hindurchführen der Schrauben 65 mit den Bezugszeichen 66 versehen. Während Figur 14 die Lage der Bohrungen 66 und damit der Schrauben 65 korrekt wiedergibt, ist in Figur 15 lediglich der besseren Übersichtlichkeit halber die Schraube 65 an anderer Stelle eingezeichnet.

Die Axialposition des Verbundes aus Abdeckplatte 47 und Seilscheibe 41 relativ zur Schraubhülse 64 wird durch eine Durchmesserstufe 67 der Schraubhülse 64 bestimmt, auf welche die Abdeckplatte 47 aufsitzt.

Die Drehlagerung der Oberschenkelschiene 6a, 6b erfolgt über einen zylindrischen Hülsenabschnitt 68 einer Mutter 69, die auf das freie Ende des Gewindeabschnitts 45 des Lagerbolzens 43 aufgeschraubt wird, bis sie an der Schraubhülse 64 anliegt. Die Mutter 69 hat dabei einen Kopfabschnitt 70, der radial über den Hülsenabschnitt 68 vorsteht und die Oberschenkelschiene 6a, 6b übergreift. Die Länge des Hülsenabschnitts 68 ist etwas größer als die Dicke der Oberschenkelschiene 6a, 6b, so dass sich die Oberschenkelschiene frei zwischen der Abdeckplatte 47 und dem Kopfabschnitt 70 drehen kann.

Aufgrund dieser Anordnung ist es möglich, mittels der Vorspannkraft der Spiralfeder 38 unabhängig von der Winkelstellung der Oberschenkelschiene 6a, 6b und Unterschenkelschiene 11a, 11b eine permanente Zugkraft auf das Zugseil 22a, 22b auszuüben, die zur Erzeugung der anhand der Figuren 9 bis 11 beschriebenen Translationskraft F in ventraler Richtung dient. Die hierzu erforderliche Federvorspannkraft wird eingestellt, indem das Antriebszahnrad 53 mittels eines geeigneten Werkzeugs gedreht wird. Hierdurch wird das Einstellzahnrad 31 und - über den Vierkantabschnitt 56 - der innere Endbereich 30 der Spiralfeder 28 entsprechend relativ zum Gehäuse 42 verdreht. Wird der äußere Endbereich 29 der Spiralfeder 28 daran gehindert, sich mitzudrehen, kann auf diese Weise je nach Drehrichtung die Einstellschraube 31 die Vorspannung der Spiralfeder 28 erhöht oder verringert werden. Dieses Festhalten des äußeren Endbereichs 29 der Spiralfeder 28 erfolgt über die Unterschenkelschiene 11a, 11b, da diese mit ihrer Mitnehmernase 63 den Schraubenkopf 61 der Mitnehmerschraube 60 hintergreift und dadurch verhindert, dass sich die Mitnehmerscheibe 58 zusammen mit der Mitnehmerschraube 60 in Vorspannrichtung dreht.

Umgekehrt bewirkt die Vorspannkraft der Spiralfeder 28, dass über das Einstellzahnrad 31 und das Antriebszahnrad 53 auf das Gehäuse 42 ein Drehmoment um die Drehachse 18 herum erzeugt wird. Dieses Drehmoment wird vom Gehäuse 42 über die Abdeckplatte 47 auf die Seilscheibe 41 übertragen. Die Vorspannkraft der Spiralfeder 28 übt somit ein Drehmoment auf die Seilscheibe 41 aus, das zu einer entsprechenden Seil- bzw. Zugkraft am Zugseil 22a, 22b führt.

Um diese Seilkraft bei Bedarf abschalten zu können, ist weiterhin eine in Figur 15 lediglich schematisch eingezeichnete Verriegelungseinrichtung 71 vorgesehen, die zwischen dem Gehäuse 42 und der Mitnehmerscheibe 58 derart wirkt, dass eine Drehbewegung der Mitnehmerscheibe 58 um die Drehachse 18 herum gesperrt werden kann. Wie aus Figur 18 ersichtlich, weist hierzu die Mitnehmerscheibe 58 an ihrem äußeren Umfang eine Verzahnung 72 auf, in die ein entsprechendes Sperrglied 73 der Verriegelungseinrichtung 71 zum Abschalten der Vorspannkraft eingeführt werden kann. Figur 15 zeigt das Sperrglied 73 in der Sperrstellung. In der Freigabestellung befindet sich das Sperrglied 73 außerhalb der Verzahnung 72.

Figur 19 zeigt eine Draufsicht auf das Gehäuse 42 mit den entsprechenden Bedienungselementen. Die Vorspannung der Spiralfeder 28 kann mit einem Inbusschlüssel eingestellt werden, der in die Sechskantaussparung 54 des Antriebszahnrads 53 eingeführt wird. Die Verriegelung und Entriegelung des Antriebszahnrads 53 erfolgt über ein Bedienelement 74 der Verriegelungseinrichtung 55. Die Verriegelung und Entriegelung der Mitnehmerscheibe 58 erfolgt über ein Bedienelement 75 der Verriegelungseinrichtung 71. Ferner weist das Gehäuse 42 im gezeigten Ausführungsbeispiel ein Fenster 76 auf, durch das hindurch Zahlen oder Markierungen ersichtlich sind, die an der Unterseite des Einstellzahnrands 31 angebracht sind und die Drehposition des Einstellzahnrads 31 relativ zum Gehäuse 42 und damit die eingestellte Vorspannkraft angeben.

Während vorstehend zwei Ausführungsbeispiele für Knieorthesen beschrieben werden, bei denen die Translationskraft F in die ventrale Richtung wirkt, kann alternativ eine in die dorsale Richtung wirkende Translationskraft auf einfache Weise dadurch geschaffen werden, dass das Schalenteil 13 auf der ventralen Seite des Unterschenkels 3 angeordnet wird und die Zugseile 22a, 22b bzw. Zugseilabschnitte 39a, 39b von den Unterschenkelschienen 11a, 11b nicht nach dorsal, sondern ventral geführt werden. Weiterhin ist es auch möglich, anstelle von zwei Zugseilen 22a, 22b ein einziges, zusammenhängendes Zugseil zu verwenden. Ferner müssen sich die Zugseile 22a, 22b nicht über eine einteilige Unterschenkelbefestigungseinrichtung 15 erstrecken, sondern können zum Beispiel auch über ein von der Unterschenkelbefestigungseinrichtung 15 getrenntes Schalenteil geführt sein.

Während bei den beschriebenen Ausführungsformen die erzeugte Translationskraft F unabhängig vom Beugewinkel des Knies ist, ist es auch ohne weiteres möglich, zusätzliche Mittel vorzusehen, mit denen die Höhe der Translationskraft F vom Beugewinkel des Knies abhängig gemacht wird, so dass sich die Translationskraft F mit sich veränderndem Beugewinkel des Knies ändert.

## Patentansprüche

1. Knieorthese zum Aufbringen einer ventral oder dorsal gerichteten Translationskraft (F) auf einen proximalen Abschnitt eines Unterschenkels (3), mit
- Oberschenkelschienen (6a, 6b), die medial und lateral am Oberschenkel (1) anordenbar und mittels einer Oberschenkelbefestigungseinrichtung (7) am Oberschenkel (1) befestigbar sind
- Unterschenkelschienen (11a, 11b), die medial und lateral am Unterschenkel (3) anordenbar und mittels einer Unterschenkelbefestigungseinrichtung (15) am Unterschenkel (3) fixierbar sind,
- einer Gelenkeinrichtung (17, 17') zur gelenkigen Verbindung der Unterschenkelschienen (11a, 11b) an den Oberschenkelschienen (6a, 6b),
- einer Translationskraftaufbringungseinrichtung zum Aufbringen der ventral oder dorsal gerichteten Translationskraft (F) auf den Unterschenkel (3),
**dadurch gekennzeichnet, dass** die Translationskraftaufbringungseinrichtung eine Seilzugeinrichtung (19, 19') mit einer Seilkrafterzeugungseinrichtung (20, 20') und einem Seilzug (21, 21') umfasst, der sich zumindest abschnittsweise von den Unterschenkelschienen (11a, 11b) nach dorsal oder ventral über einen proximalen Abschnitt des Unterschenkels (3) quer zur Längserstreckung der Unterschenkelschienen (11a, 11b) erstreckt, an den Unterschenkelschienen (11a, 11b) befestigt oder umgelenkt ist und mittels einer von der Seilkrafterzeugungseinrichtung (20, 20') in den Seilzug (21, 21') eingeleiteten Seilkraft den proximalen Abschnitt des Unterschenkels (3) in die ventrale oder dorsale Richtung drängt.

2. Knieorthese nach Anspruch 1, **dadurch gekennzeichnet, dass** der Seilzug (21) zwei Zugseile (22a, 22b) umfasst, wobei ein erstes Zugseil (22a) an der lateralen Unterschenkelschiene (11a) und ein zweites Zugseil (22b) an der medialen Unterschenkelschiene (11b) befestigt ist und die Seilkrafterzeugungseinrichtung (20) ein zentrales Seilkrafterzeugungsmittel ist, mit dem beide Zugseile (22a, 22b) in Wirkverbindung sind.

3. Knieorthese nach Anspruch 1 oder 2, **dadurch gekennzeichnet, dass** die Seilkrafterzeugungseinrichtung (20) auf einem zur Anlage am Unterschenkel (3) bestimmten und relativ zu den Unterschenkelschienen (11a, 11b) bewegbaren Schalenteil (13) befestigt ist.

4. Knieorthese nach Anspruch 3, **dadurch gekennzeichnet, dass** das Schalenteil (13) ein proximaler Teilabschnitt der Unterschenkelbefestigungseinrichtung (15) ist, der relativ zu den Unterschenkelschienen (11a, 11b) in Dorsaler und ventraler Richtung schwenkbar ist.

5. Knieorthese nach Anspruch 1, **dadurch gekennzeichnet, dass** der Seilzug (21') mindestens ein Zugseil (22a, 22b) umfasst, das einen ersten Zugseilabschnitt (37a, 37b), der längs einer Unterschenkelschiene (11a, 11b) verläuft, und einen zweiten Zugseilabschnitt (39a, 39b) aufweist, der sich quer zu dieser Unterschenkelschiene (11a, 11b) nach dorsal oder ventral über den Unterschenkel (3) erstreckt und mittels einer an der Unterschenkelschiene (11a, 11b) angeordneten Seilumlenkeinrichtung (38a, 38b) umgeleitet wird, wobei die Seilkrafterzeugungseinrichtung (20') im Bereich der Gelenkeinrichtung (17') oder Unterschenkelschiene (11a, 11b) angeordnet ist.

6. Knieorthese nach Anspruch 5, **dadurch gekennzeichnet, dass** der Seilzug (21') ein erstes Zugseil (22a) und ein zweites Zugseil (22b) umfasst, wobei das erste Zugseil (22a) einen im Bereich der lateralen Unterschenkelschiene (11a) angeordneten ersten Zugseilabschnitt (37a) und das zweite Zugseil (22b) einen im Bereich der medialen Unterschenkelschiene (11b) angeordneten ersten Zugseilabschnitt (37b) aufweisen, und wobei die Seilkrafterzeugungseinrichtung (20') ein erstes Seilkrafterzeugungsmittel (20a), das lateral im Bereich der Gelenkreinrichtung (17') oder der lateralen Unterschenkelschiene (11a) angeordnet ist, und ein zweites Seilkrafterzeugungsmittel (20b) umfasst, das medial im Bereich der Gelenkeinrichtung (17') oder der medialen Unterschenkelschiene (11b) angeordnet ist.

7. Knieorthese nach Anspruch 5 oder 6, **dadurch gekennzeichnet, dass** sich die zweiten Zugseilabschnitte (39a, 39b) über ein zur Anlage am Unterschenkel (3) bestimmtes und relativ zu den Unterschenkelschienen (11a, 11b) bewegbares Schalenteil (13) erstrecken.

8. Knieorthese nach Anspruch 7, **dadurch gekennzeichnet, dass** das Schalenteil (13) ein proximaler Teilabschnitt der Unterschenkelbefestigungseinrichtung (15) ist, der relativ zu den Unterschenkelschienen (11a, 11b) nach dorsal und ventral schwenkbar ist.

9. Knieorthese nach einem der Ansprüche 2 bis 8, **dadurch gekennzeichnet, dass** die Seilkrafterzeugungseinrichtung (20, 20') eine drehbare Seilscheibe (25, 41) umfasst, an der das mindestens eine Zugseil (22a, 22b) außermittig befestigt ist, wobei die Seilscheibe (25, 41) mittels einer Federeinrichtung in eine bestimmte Drehrichtung vorgespannt ist.

10. Knieorthese nach Anspruch 9, **dadurch gekennzeichnet, dass** die Federeinrichtung eine Spiralfeder (28) umfasst, die einen ersten Endbereich (30), der mit einem Einstellzahnrad (31) gekoppelt ist, und einen zweiten Endbereich (29) aufweist, der mit der Seilscheibe (25, 41) gekoppelt ist, wobei die Vorspannung der Seilscheibe (25, 41) durch Drehen des Einstellzahnrads (31) variierbar ist.
